(19) Europäisches Patentamt
European Patent Office
Office européen des brevets

(11) EP 2 626 358 B1

(12) EUROPEAN PATENT SPECIFICATION

(45) Date of publication and mention of the grant of the patent:
**02.09.2015 Bulletin 2015/36**

(51) Int Cl.:
***C07D 405/06*** *(2006.01)* ***C07K 16/44*** *(2006.01)*
***G01N 33/94*** *(2006.01)*

(21) Application number: **13154614.5**

(22) Date of filing: **08.02.2013**

(54) **Immunoassay for pyrrolidinophenones**

Immunoassay für Pyrrolidinophenone

Dosage immunologique pour pyrrolidinophenones

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2012 GB 201202223**

(43) Date of publication of application:
**14.08.2013 Bulletin 2013/33**

(73) Proprietor: **Randox Laboratories Ltd.**
**Crumlin, County Antrim BT29 4QY (GB)**

(72) Inventors:
- **Lowry, Philip**
  **Crumlin, Antrim BT29 4QY (GB)**
- **Benchikh, Elouard**
  **Crumlin, Antrim BT29 4QY (GB)**
- **McConnell, Ivan**
  **Crumlin, Antrim BT29 4QY (GB)**
- **Fitzgerald, Peter**
  **Crumlin, Antrim BT29 4QY (GB)**

(56) References cited:
- **STRANO-ROSSI S. ET AL.: RAPID COMMUN. MASS SPECTROM., vol. 24, 2010, pages 2706-2714, XP002696686,**
- **PIRKKO KRIIKKU ET AL: "New designer drug of abuse: 3,4-Methylenedioxypyrovalerone (MDPV). Findings from apprehended drivers in Finland", FORENSIC SCIENCE INTERNATIONAL, ELSEVIER SCIENTIFIC PUBLISHERS IRELAND LTD, IE, vol. 210, no. 1, 12 March 2011 (2011-03-12), pages 195-200, XP028232874, ISSN: 0379-0738, DOI: 10.1016/J.FORSCIINT. 2011.03.015 [retrieved on 2011-03-21]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 626 358 B1

## Description

## BACKGROUND TO THE INVENTION

The invention relates to the field of analytical detection of pyrrolidinophenones. The pyrrolidinophenones, typically classified as a sub-set of the synthetic cathinones because of their structural similarities, represent a recent class of psychoactive drug comprising the following structure (also referred to as a 'sub-structure').

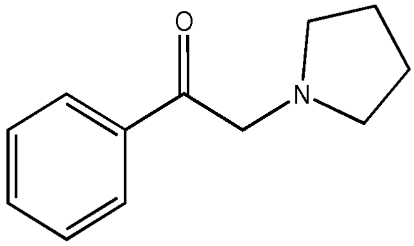

Structure I

Several pyrrolidinophenones have been described which possess psychoactive properties including (RS)-1-(benzo[d][1,3]dioxol-5-yl)-2-(pyrrolidin-1-yl)pentan-1-one (commonly referred to as 3,4-methylenedioxypyrovalerone or MDPV, molecule 25 of Figure 1) and (RS)-1-(4-methylphenyl)-2-(1-pyrrolidinyl)pentan-1-one (commonly referred to as pyrovalerone or PVP, molecule 7 of Figure 1). Figure 1 lists known pyrrolidinophenones with psychoactive properties and proposed metabolites. Their ingestion produces feelings of euphoria, induces intoxication-like behaviour which affects the ability to drive and can result in death. There is a need to detect pyrrolidinophenones both pre and post ingestion for toxicological and legal purposes, several molecules of the class being either illegal or undergoing legal scrutiny in various jurisdictions world-wide. They are marketed under various guises such as plant food, research chemicals and bath salts often explicitly warning against human consumption, an approach probably designed to circumvent statutory restrictions for these substances. Current screening and confirmation methods use mass-spectrometry (MS) in conjunction with one or more of gas chromatography (GC), liquid chromatography (LC), nuclear magenetic resonance (NMR) and Fourier transform infrared spectroscopy (FTIR) to detect MDPV, its metabolites and other pyrrolidinophenones (e.g.Yohannan and Bozenko 2010; Strano-rossi et al. 2010). These methods are resource intensive requiring expensive equipment and highly trained staff for their operation. Furthermore, the high number of known, possibly unknown (i.e. pyrrolidinophenones being used but yet to be characterised) and future psychoactive pyrrolidinophenone drugs results in compounds with different MS fragmentation patterns leading to difficulties and uncertainty with their identification using established methods. Thus what is required is a simplistic and economical detection method that is able to tackle the extensive and growing number of psychoactive substances of the pyrrolidinophenone class.

## References

Yohannan J.C. and Bozenko J.S. (2010). Microgram J., 1:12-15.
Strano-Rossi S. et al. (2010). Rapid Commun. Mass Spectrom., 24:2706-2714.
Fitzgerald S.P. et al. (2005). Clin. Chem., 51: 1165-1176.

## DRAWINGS

Figure 1. Table of psychoactive pyrollidinophenones and metabolites (Italicised)
Figure 2. Synthesis of Hapten A
Figure 3. Synthesis of Hapten B
Figure 4. C-13 NMR of Hapten A
Figure 5. C-13 NMR of Hapten B
Figure 6. MDPBP dose-response calibration curve used to determine antibody sensitivity and specificity to psychoactive pyrrolidinophenones and other psychoactive compounds

## SUMMARY OF THE INVENTION

Described herein is the first known immunoassay for the detection of pyrrolidinophenones. The immunoassay is an inexpensive, relatively simplistic analytical technique. The invention describes antibodies, derived from novel haptens (pre-immunogenic molecules) and immunogens, whose binding properties enable the detection of several psychoactive pyrrolidinophenones. The haptens, before conjugation to a crosslinking group, are uniquely in possession of a hydroxyl group at the 3-position of the pyrrolidone ring. This hydroxyl group can be attached, using standard reagents and techniques, initially to a crosslinking molecule prior to attachment to the antigenicity conferring carrier material (accm) to form the immunogens of the invention. Also described are novel methods, kits and uses each comprising antibodies of the invention. What is particularly surprising and beneficial about this immunoassay is the high number of psychoactive pyrrolidinophenones of diverse structure that the antibodies are able to bind, providing for an immunoassay of great scope and utility.

## DETAILED DESCRIPTION OF THE INVENTION

In a first aspect the invention describes haptens and immunogens of Formula I

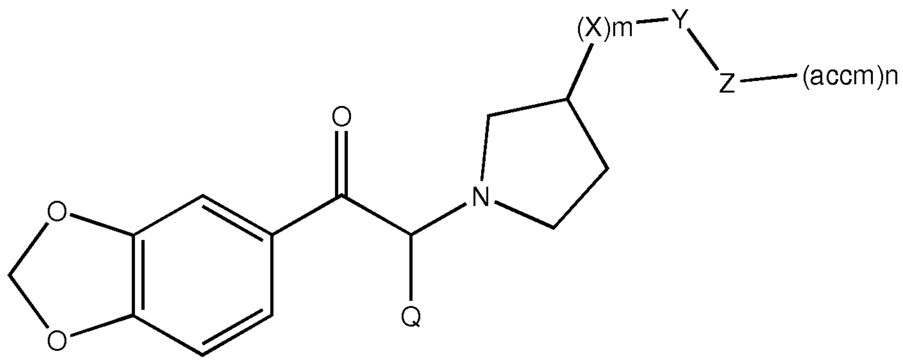

Formula I

wherein,

for the haptens n=0; Q is $C_1$ - $C_4$ alkyl; X is N, O or S and m=0 or 1; Y is a substituted or unsubstituted $C_1$ - $C_{10}$, more preferably a $C_1$ - $C_6$, most preferably a $C_1$ - $C_3$ substituted or unsubstituted straight chain alkylene moiety; Z is selected from a carboxy, a dithiopyridyl, a maleimide, amino, hydroxyl, thiol or an aldehyde moiety,

and for the immunogens n=1; Q is $C_1$ - $C_4$ alkyl; X is N, O or S and m=0 or 1; Y is a substituted or unsubstituted $C_1$ - $C_{10}$, more preferably a $C_1$ - $C_6$, most preferably a $C_1$ - $C_3$ substituted or unsubstituted straight chain alkylene moiety; Z, before connection to the accm, is selected from a carboxy, a dithiopyridyl, a maleimide, amino, hydroxyl, thiol or an aldehyde moiety; the accm is an antigenicity-conferring carrier material.

For both the haptens and immunogens it is preferable that X is O, Y is $-C(O)-CH_2-CH_2-$ , Z is carboxy or amino (for the immunogen, Z is carboxy or amino before connection to the accm) and Q is preferably a straight chain alkyl most preferably either ethyl or propyl. The immunogens are prepared by coupling a hapten (a pre-immunogenic molecule) to an antigenicity-conferring carrier material (accm) usually by way of a crosslinker. The accm and the crosslinkers are standard reagents in the field of antibody development. The accm comprises polyamino acid segments and is preferably bovine thyroglobulin (BTG), bovine serum albumin (BSA) or keyhole limpit haemocyanin (KLH). Alternatively, if it has a suitable functional group, the hapten may be attached directly to the accm without the use of a crosslinker. An example of a crosslinker described herein is succinic anhydride, activated by N,N-dicyclohexylcarbodiimide and N-hydroxysuccinimide.

A second aspect of the invention is antibodies raised to immunogens of Formula I, the antibodies being capable of binding to at least one structural epitope of a molecule of Formula II

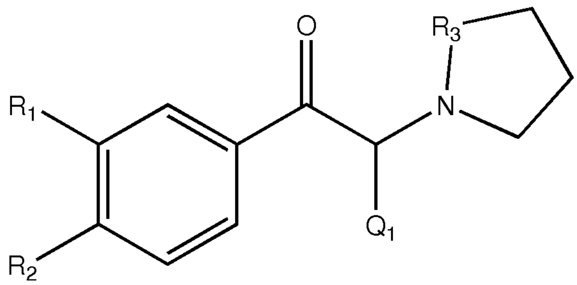

Formula II

wherein

$Q_1$ is $C_1$ - $C_4$ alkyl; $R_3$ is $-CH_2-$ or -C(O)- ; $R_1$ and $R_2$ are H, $C_1$ - $C_4$ alkyl,$C_1$ - $C_4$ alkoxy, hydroxy substituted $C_1$ - $C_4$ alkyl, carboxy or hydroxyl, or together form substituted or unsubstituted

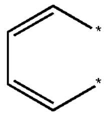

or

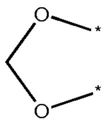

to form a fused bicyclic ring with the benzene ring. In preferred embodiments the at least one structural epitope of Formula II bound by the antibodies are when the fused bicyclic ring is unsubstituted, $Q_1$ is methyl, ethyl or propyl and $R_3$ is $-CH_2-$ . The antibodies are obtained using standard methods; immunogens of the invention are administered to a non-human mammalian host, preferably a sheep, to elicit antibody production after which harvested polyclonal or monoclonal antibodies are used to develop immunoassays. Other suitable immunoglobulin-derived molecules such as short-chain or single chain variable fragments are readily applied alternatives known to the skilled person. Examples of pyrrolidinophenones which are bound by the antibodies of the invention are shown in Figure 1. In a preferred embodiment the antibodies of the invention are able to bind to at least one structural epitope of the molecules (RS)-1-( benzo[d][1,3] dioxol-5-yl)-2-(pyrrolidin-1-yl)butanone (common name MDPBP), (RS)-1-(2-naphthyl)-2-(pyrrolidin-1-yl)pentanone (common name naphyrone), (RS)-1-(4-methylphenyl)-2-(pyrrolidin-1-yl)butanone, (RS)-1-(benzo[d][1,3]dioxol-5-yl)-2-(pyrrolidin-1-yl)pentanone or (RS)-1-(benzo[d][1,3]dioxol-5-yl)-2-(pyrrolidin-1-yl)propanone (common name MDPPP); all five drugs incorporate Structure I in their molecular structure. It is known in the art that for two different molecules to be able to independently bind to a particular protein their structures must be similar; any substantial deviation in structure results in a molecule with a markedly reduced affinity for the protein. This imposes a limit on the number of molecules that have substantial binding affinity for a particular protein. The pyrrolidinophenones are thought to bind to the monoamine transporter proteins for serotonin, dopamine and noradrenaline, increasing the concentration of amines in the central nervous system resulting in stimulatory and hallucinogenic effects. Currently known psychoactive pyrrolidinophenones are listed in Figure 1.

A further aspect of the invention is a method of detecting or determining one or more pyrrolidinophenones in an in vitro sample comprising a substance (the substance optionally having been pre-treated to attain a suitable state for analysis) the method comprising contacting the sample with at least one detecting agent and at least one antibody of the invention; detecting or determining the detecting agent(s); and deducing from a calibration curve the presence of, or amount of, pyrrolidinophenones in the sample.

Preferably the pyrrolidinophenones to be detected or determined are listed in Figure 1; most preferably the one or more pyrrolidinophenones to be detected or determined is/are (RS)-1-(benzo[d][1,3]dioxol-5-yl)-2-(pyrrolidin-1-yl)butanone, (RS)-1-(2-naphthyl)-2-(pyrrolidin-1-yl)pentanone, (RS)-1-(4-methylphenyl)-2-(pyrrolidin-1-yl)butanone, (RS)-1-(benzo[d][1,3]dioxol-5-yl)-2-(pyrrolidin-1-yl)pentanone and (RS)-1-(benzo[d][1,3]dioxol-5-yl)-2-(pyrrolidin-1-yl)propanone. The detecting agent comprises a suitable hapten, preferably the haptens disclosed herein, covalently bonded to a detectable labelling agent, the hapten moiety being able to bind to the antibodies of the invention. Preferably, the labelling agent is selected from an enzyme, a luminescent substance, a radioactive substance, or a mixture thereof. More preferably, the labelling agent is an enzyme, preferably a peroxidase, most preferably horseradish peroxidise (HRP). Detecting implies qualitatively analyzing for the presence or absence of a substance; determining means quantitatively analyzing the amount of substance. As the antibodies are able to bind to several molecules, quantitative analysis will take the form of measuring the calibrator-equivalent amount. The main use of the pyrrolidinophenone immunoassay described herein, as with most immunoassays, is perceived to be as a screening tool in which target molecules are

detected and subsequently identified using mass-spectrometry based methods. Any suitable in vitro biological sample may be used, but blood and urine are preferred.

A further aspect of the invention is a compound of Formula III

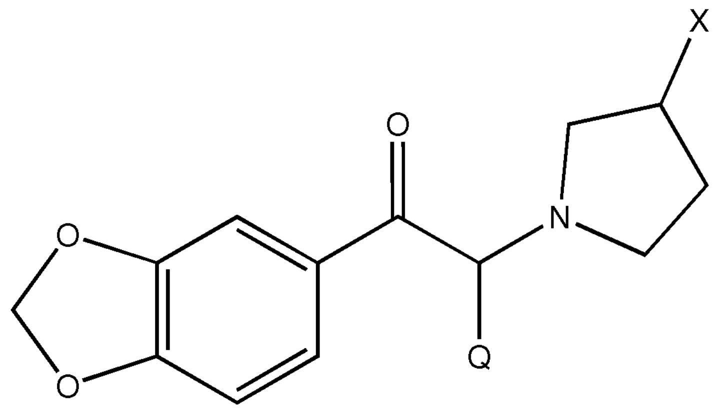

Formula III

in which X is $NH_2$, OH or SH and Q is $C_1$ - $C_4$ alkyl.

In preferred embodiments X is OH and Q is ethyl or propyl. It is also preferable that the asymmetric carbon atom of the heterocyclic ring of Formula III is stereospecifically of the R configuration. Compounds of Formula III are representative haptenic intermediate molecules which can be conjugated to crosslinking reagents prior to immunogen formation.

Another aspect of the invention is a kit for detecting or determining pyrrolidinophenones the kit comprising at least one antibody of the invention. The kit preferably detects or determines one or more pyrrolidinophenones listed in Figure 1. Most preferably the kit detects or determines (RS)-1-( benzo[d][1,3]dioxol-5-yl)-2-(pyrrolidin-1-yl)butanone, (RS)-1-(2-naphthyl)-2-(pyrrolidin-1-yl)pentanone, (RS)-1-(4-methylphenyl)-2-(pyrrolidin-1-yl)butanone, (RS)-1-(benzo[d][1,3]dioxol-5-yl)-2-(pyrrolidin-1-yl)pentanone and (RS)-1-(benzo[d][1,3]dioxol-5-yl)-2-(pyrrolidin-1-yl)propanone. The kit may comprise one or more antibodies of the invention and one or more additional antibodies with different molecular specificities i.e. these additional antibodies do not bind to the same structural epitopes as the antibodies of the invention. Such an arrangement enables a multiplex approach to the detection or determination of drugs of abuse. The multiplex approach preferably makes use of a planar substrate to which the antibodies are attached, such as a ceramic chip or an appropriately surface-modified glass slide. Beads may also be used as a substrate in a singleplex or multiplex approach.

**General Methods, Examples and Results**

Preparation of Haptens, Immunogens and Detecting Agents

Although haptens provide defined structural epitopes, they are not in themselves immunogenic and therefore need to be conjugated to an antigenicity conferring carrier material (accm) which will elicit an immunogenic response when administered to a host animal. Appropriate accms commonly contain poly(amino acid) segments and include polypeptides, proteins and protein fragments. Illustrative examples of useful carrier materials are bovine serum albumin (BSA), egg ovalbumin, bovine gamma globulin, BTG, keyhole limpet haemocyanin (KLH) etc. Alternatively, synthetic poly(amino acids) having a sufficient number of available amino groups, such as lysine, may be employed, as may other synthetic or natural polymeric materials bearing reactive functional groups. Also, carbohydrates, yeasts or polysaccharides may be conjugated to the hapten to produce an immunogen. The haptens can also be coupled to a detectable labelling agent such as an enzyme (for example, horseradish peroxidase), a substance having fluorescent properties or a radioactive label for the preparation of detecting agents for use in the immunoassays. The fluorescent substance may be, for example, a monovalent residue of fluorescein or a derivative thereof. Immunogen formation for the invention described herein involves conventional conjugation chemistry. In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS).

General Procedure for MALDI-TOF Analysis of Immunogens

MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1μl) were analysed using a matrix of sinapinic acid and bovine serum albumin (Fluka) was used as an external calibrant.

Preparation of Antisera

In order to generate polyclonal antisera, an immunogen of the present invention is mixed with Freund's adjuvant and the mixture is injected into a host animal, such as rabbit, sheep, mouse, guinea pig or horse. Sheep are the preferred host animal. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate non-specific binding.

Immunoassay Development

The process of developing an immunoassay is well known to the person skilled in the art. Briefly, for a competitive immunoassay in which the target analyte is a non-immunogenic molecule such as a hapten, the following process is conducted: antibodies are produced by immunising an animal, preferably a mammalian animal, by repeated administration of an immunogen. The serum from the immunised animal is collected when the antibody titre is sufficiently high. A detecting agent is added to a sample containing the target analyte and the raised antibodies, and the detecting agent and analyte compete for binding to the antibodies. The process may comprise fixing said serum antibodies to a backing substrate such as a polystyrene solid support or a ceramic chip. The antibodies can be polyclonal or monoclonal using standard techniques. The signal emitted in the immunoassay is proportionate to the amount of detecting agent bound to the antibodies which in turn is inversely proportionate to the analyte concentration. The signal can be detected or quantified by comparison with a calibrator.

**Examples**

Example 1: Synthesis of (RS)-1-(benzo[d][1,3]dioxol-5-yl)-2-bromobutanone

To a solution of 1-(benzo[d][1,3]dioxol-5-yl)butanone (25g, 0.13 mole) in acetic acid (100ml) was added dropwise a solution of bromine (21.8g, 0.137 mole) in acetic acid (100ml). The reaction mixture was then stirred at room temperature for two hours. Acetic acid was removed under high vacuum. Water (200ml) was added to the mixture and the solution was extracted with dichloromethane (2x200m1). The combined organic layers were washed with saturated $NaHCO_3$ solution (100ml), water (100ml) and brine (100ml). The dichloromethane solution was dried over $Na_2SO_4$, filtered and concentrated to dryness under vacuum. The crude product obtained was purified by chromatography on silica gel using 5% ethyl acetate in hexane to give the title compound (29.6g, 84%).

Example 2: Synthesis of (RS)-1-(benzo[d][1,3]dioxol-5-yl)-2-((R)-3-hydroxypyrrolidin-1-yl) butanone

To a solution (RS)-1-(benzo[d][1,3]dioxol-5-yl)-2-bromobutanone (7.92g, 27.8mmol) in acetonitrile (100ml) was added potassium carbonate (7.93g, 57.4mmol) and (R)-(+)-3-pyrrolidinol (5.0g, 57.4mmol) and the mixture stirred under nitrogen overnight at room temperature. The mixture was filtered and the solution evaporated to dryness. The crude obtained was purified by chromatography on silica gel using 50% ethyl acetate in hexane to give the title compound as a brown oil (5.5g, 69.2%).

Example 3: Synthesis of Hapten-A

To a solution of 1-(Benzo[d][1,3]dioxol-5-yl)-2-((R)-3-hydroxypyrrolidin-1-yl)butanone (4.5g, 16.25mmol) in anhydrous pyridine (100ml) was added succinic anhydride (3.25g, 32.5mmol) and the mixture stirred overnight at room temperature. The pyridine was removed under high vacuum and the dark brown crude obtained purified by chromatography on silica gel using 20% methanol in chloroform to give Hapten - A (Figure 2) as a light tan oil (5.95g, 97.1%).

Example 4: Conjugation of Hapten-A to BSA (Immunogen I)

To a solution of Hapten-A (42.62mg, 0.113mmol) in DMF (1.0ml) was added N, N-dicyclohexylcarbodiimide (DCC) (25.64mg, 0.125mmol) and N-hydroxysuccinimide (14.3mg, 0.16mM) and the mixture stirred at room temperature overnight. The dicyclohexylurea formed was removed by filtration and the solution was added dropwise to a solution of BSA (150mg, 2.3μmol) in 50mM sodium bicarbonate solution (pH 8.5) (10ml). The mixture was then stirred overnight at 4°C. The solution was dialysed against 50mM phosphate buffer pH 7.2 (3 changes) for 24 hours at 4°C, and freeze-dried to give Immunogen I.
MALDI results showed 16.58 molecules of Hapten-A had been conjugated to one molecule of BSA.

Example 5: Conjugation of Hapten-A to BTG (Immunogen II)

To a solution of Hapten-A (50.94mg, 0.135mmol) in DMF (1.0ml) was added N, N-dicyclohexylcarbodiimide (DCC) (30.64mg, 0.150mmol) and N-hydroxysuccinimide (17.1mg, 0.15mmol) and the mixture stirred at room temperature overnight. The dicyclohexylurea formed was removed by filtration and the solution was added dropwise to a solution of BTG (150mg, 2.25 μmol) in 50mM sodium bicarbonate solution (pH 8.5) (10ml). The mixture was then stirred overnight at 4°C. The solution was dialysed against 50mM phosphate buffer pH 7.2 (3 changes) for 24 hours at 4°C, and freeze-dried to give Immunogen II.

Example 6: Conjugation of Hapten-A to HRP

EDC hydrochloride (10mg) was dissolved in water (0.5ml) and immediately added to a solution of hapten -A (2mg) in DMF (0.2ml). After mixing, the solution was added dropwise to a solution of HRP (20mg) in water (1ml). Sulfo-NHS (5mg) was added and the reaction mixture incubated in the dark at room temperature overnight. Excess hapten was removed with double PD-10 columns (Pharmacia) in series, pre-equilibrated with PBS at pH 7.2. The hapten-HRP conjugate was then dialysed overnight against 10L of PBS at pH 7.2 at 4°C.

Example 7: Synthesis of (RS)-1-(benzo[d][1,3]dioxol-5-yl)-2-bromopentanone

To a solution of 1-(benzo[d][1,3]dioxol-5-yl)pentanone (25g, 0.12 mole) in acetic acid (100ml) was added dropwise a solution of bromine (25.2g, 0.158 mole) in acetic acid (100ml). The reaction mixture was then stirred at room temperature for two hours. Acetic acid was removed under high vacuum. Water (200ml) was added to the mixture and the solution extracted with dichloromethane (2x200ml). The combined organic layers were washed with saturated $NaHCO_3$ solution (100ml), water (100ml) and brine (100ml). The dichloromethane solution was dried over $Na_2SO_4$, filtered and concentrated to dryness under vacuum. The crude product obtained was purified by chromatography on silica gel using 5% ethyl acetate in hexane to give the title compound (30.5g, 89%).

Example 8: Synthesis of (RS)-1-(benzo[d][1,3]dioxol-5-yl)-2-((R)-3-hydroxpyrrolidin-1-yl)pentanone

To a solution (RS)-1-(benzo[d][1,3]dioxol-5-yl)-2-bromopentanone (10.0g, 35.07mmol) in acetonitrile (100ml) was added potassium carbonate (10.0g, 72.41mmol) and (R)-(+)-3-pyrrolidinol (6.3g, 72.4mmol) and the mixture stirred under nitrogen overnight at room temperature. The mixture was then filtered and the solution evaporated to dryness. The crude obtained was purified by chromatography on silica gel using 50% ethyl acetate in hexane to give the title compound as a brown foamy solid (6.1g, 60.0%).

Example 9: Synthesis of Hapten-B

To a solution of (RS)-1-(benzo[d][1,3]dioxol-5-yl)-2-((R)-3-hydroxypyrrolidin-1-yl)pentanone (5.8g, 20.0mmol) in anhydrous pyridine (100ml) was added succinic anhydride (3.0g, 30.0mmol) and the mixture was stirred overnight at room temperature. The pyridine was removed under high vacuum and the dark brown crude obtained was purified by chromatography on silica gel using 20% methanol in chloroform to give the pure Hapten-B as a tan solid (5.3g, 68.0%).

Example 10: Conjugation of Hapten-B to BSA (Immunogen-III)

To a solution of Hapten-B (35.22mg, 0.09mmol) in DMF (1.0ml) was added N, N-dicyclohexylcarbodiimide (DCC) (20.42mg, 0.099mmol) and N-hydroxysuccinimide (11.39mg, 0.099mmol) and the mixture was stirred at room temperature overnight. The dicyclohexylurea formed was removed by filtration and the solution was added dropwise to a solution of BSA (150mg, 2.3μmol) in 50mM sodium bicarbonate solution (pH 8.5) (10ml). The mixture was then stirred

overnight at 4°C. The solution was dialysed against 50mM phosphate buffer pH 7.2 (3 changes) for 24 hours at 4°C, and freeze-dried to give Immunogen-III. MALDI results showed 19.63 molecules of Hapten-B had been conjugated to one molecule of BSA.

Example 11: Conjugation of Hapten-B to BTG (Immunogen-IV)

To a solution of Hapten - B (44.22mg, 0.113mmol) in DMF (1.0ml) was added N, N-dicyclohexylcarbodiimide (DCC) (25.64mg, 0.13mmol) and N-hydroxysuccinimide (14.7mg, 0.13mmol) and the mixture was stirred at room temperature overnight. The dicyclohexylurea formed was removed by filtration and the solution was added dropwise to a solution of BTG (150mg, 2.25μmol) in 50mM sodium bicarbonate solution (pH 8.5) (10ml). The mixture was then stirred overnight at 4°C. The solution was dialysed against 50mM phosphate buffer pH 7.2 (3 changes) for 24 hours at 4°C, and freeze-dried to give Immunogen-IV.

Example 12: Conjugation of Hapten-B to HRP

EDC hydrochloride (10mg) was dissolved in water (0.5ml) and immediately added to a solution of hapten -B (2mg) in DMF (0.2ml). After mixing, this solution was added dropwise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP conjugate was then dialysed overnight against 10L of PBS at pH 7.2 at 4° C.

Example 13: Immunoassay of pyrrolidinophenones and selected molecules

A semi-automated Evidence Investigator analyser (Randox Laboratories Ltd., Crumlin, UK) was used as the platform for a biochip assay for the detection of pyrrolidinophenones. Immunogen IV was administered to adult sheep on a monthly basis to provide target-specific polyclonal antisera. IgG was extracted from the antisera, and the purified antibody was immobilised on a biochip (9mm x 9mm). The assay is based on competition for binding sites of a polyclonal antibody between Hapten-A conjugate (Example 6) and pyrrolidinophenones and potential cross-reactants. The antibody was immobilised and stabilised onto the biochip surface as previously described (Fitzgerald et al., 2005). Assay diluent (155μL), calibrator/pyrrolidinophenone or potential cross-reactant (25μL) followed by Hapten-A conjugate (120μL) were added to the appropriate biochip. The biochips were then incubated for 30 minutes at 30°C on a thermoshaker set at 370 rpm. The biochips were then subjected to 2 quick wash cycles using the wash buffer provided, followed by 4 x 2 minute wash cycles. 250μL of signal (1:1 luminol + peroxide, v/v) was then added to each biochip, and after 2 minutes the biochip carrier was imaged in the Evidence Investigator analyser. Calibration curves were generated and these were used to determine the sensitivity and specificity of the immunoassay for pyrrolidinophenones and potential cross-reactants. The results of this study are presented in Table 1, cross-reactivity being calculated according to the following formula:

$$\%CR = IC_{50\ MDPBP} / IC_{50\ CR} \times 100$$

where %CR is the percentage cross-reactivity, $IC_{50\ MDPBP}$ is the concentration of MDPBP which causes 50% displacement of signal and $IC_{50\ CR}$ is the concentration of pyrrolidinophenone/potential cross-reactant that causes 50% displacement of signal.

Chemicals

MDPBP HCl, MDPV HCl, (RS)-1-(4-methylphenyl)-2-(pyrrolidin-1-yl)butanone HCl, naphyrone HCL, and MDPPP HCl were obtained from the Australian Government National Measurement Institute (LGC stds); MDMA and MDA were obtained from Cerilliant; amphetamine, methamphetamine HCl and JWH-018 were obtained from Sigma Chemicals; mescaline HCl and (+)-pseudoephedrine were obtained from Sigma Aldrich; MDMA and MDA were obtained from Cerilliant; 1-(3-chlorophenyl)piperazine was obtained from Alfa Aesar; salvinorin A and 1-benzylpiperazine were obtained from Aaron Chemistry; JWH-250 was obtained from Cayman Chemicals; mephedrone HCl was synthesised at Randox Laboratories.

Results

**Table 1** Cross-reactivity results for pyrrolidinophenones and potential cross-reactants ($IC_{50}$ of (RS)-1-(benzo[d][1,3] dioxol-5-yl)-2-(pyrrolidin-1-yl)butanone is 27 ng/ml)

| Substance | % Cross-Reactivity |
|---|---|
| (RS)-1-(Benzo[d][1,3]dioxol-5-yl)-2-(pyrrolidin-1-yl)butanone HCl | 192 |
| (RS)-1-(2-Naphthyl)-2-(pyrrolidin-1-yl)pentanone HCl | 44 |
| (RS)-1-(4-Methylphenyl)-2-(pyrrolidin-1-yl)butanone HCl | 34 |
| (RS)-1-(Benzo[d][1,3]dioxol-5-yl)-2-(pyrrolidin-1-yl)pentanone HCl | 100 |
| (RS)-1-(Benzo[d][1,3]dioxol-5-yl)-2-(pyrrolidin-1-yl)propanone HCl | 12 |
| (RS)-1-(Benzo[d][1,3]dioxol-5-yl)-N-methylpropan-2-amine (MDMA) | <1 |
| D-Amphetamine | <1 |
| (RS)-1-(benzo[d][1,3]dioxol-5-yl)propan-2-amine (MDA) | <1 |
| Methamphetamine HCl | <1 |
| Mescaline HCl | <1 |
| (+)-Pseudoephedrine HCl | <1 |
| Salvinorin A | <1 |
| JWH-018 | <1 |
| JWH-250 | <1 |
| 1-Benzylpiperazine | <1 |
| 1-(3-Chlorophenyl)piperazine HCl | <1 |
| Mephedrone HCl | <1 |

The data in Table 1 confirms that antibodies of the invention bind to pyrrolidinophenones incorporating the sub-structure depicted in Structure I while not binding to molecules lacking this sub-structure.

**Table 2** Further cross-reactivity results for pyrrolidinophenones and potential cross-reactants

| **Compound** | **% Cross-reactivity** |
|---|---|
| (RS)-1-(Benzo[d][1,3]dioxol-5-yl)-2-(pyrrolidin-1-yl)pentanone HCl | 100 |
| (RS)-1-(4-Methylphenyl)-2-(pyrrolidin-1-yl)hexanone HCl | 48.4 |
| Pyrovalerone HCl | 44.4 |
| Pentylone HCl | 17.4 |
| Butylone HCl | 11.4 |
| Pentedrone HCl | < 1 |
| Methylone HCl | < 1 |
| Nor-mephedrone HCl | < 1 |
| 1-Phenyl-2(1-pyrrolidinyl)ethanone | < 1 |
| 1,2 Dihydroxybenzene (Catechol) | < 1 |
| N,N-diallyl-5-methoxytryptamine (5-MeO-DALT) | < 1 |
| Dihydroxymephedrone HCl | < 1 |
| Flephedrone HCl | < 1 |

(continued)

| Compound | % Cross-reactivity |
|---|---|
| Prolatine | < 1 |
| Heroin | < 1 |
| Acetaminophen | < 1 |
| Buprenorphine | < 1 |
| Bupropion HCl | < 1 |
| 1-Benzyl-4-methylpiperazine HCl monohydrate | < 1 |
| BZG | < 1 |
| (-)-Cotinine | < 1 |
| Dextromethorphan hydrobromide monohydrate | < 1 |
| Diethylpropion HCl | < 1 |
| Ethyl-glucuronide | < 1 |
| Fentanyl | < 1 |
| Fluoxetine HCl | < 1 |
| 7-NH Flunitrazepam | < 1 |
| Haloperidol | < 1 |
| Hydrocodone | < 1 |
| Hydromorphone | < 1 |
| Ibuprofen | < 1 |
| Indole-3-carboxylic acid | < 1 |
| Lorazepam | < 1 |
| LSD | < 1 |
| Meprobamate | < 1 |
| Methadone | < 1 |
| Methaqualone | < 1 |
| Methylphenidate HCl | < 1 |
| Morphine sulphate | < 1 |
| (-)-Nicotine | < 1 |
| (+)-Norpropoxyphene maleate | < 1 |
| (±)-Norketamine HCl | < 1 |
| Norescitalopram | < 1 |
| Normeperidine | < 1 |
| Nortriptyline HCl | < 1 |
| Oxazepam | < 1 |
| Oxycodone | < 1 |
| 1-(1-phenylcyclohexyl)piperidine) HCl (PCP HCl) | < 1 |
| Phenobarbital | < 1 |
| Phenethylamine sulphate | < 1 |
| Psilocin | < 1 |
| Salicylic acid | < 1 |
| Salicyluric acid | < 1 |
| Sertraline HCl | < 1 |
| (-)-11-nor-9-Carboxy-delta9-THC | < 1 |
| (-)-Delta9-THC | < 1 |
| Tramadol HCl | < 1 |
| Trazadone HCl | < 1 |
| (+/-)-3,4,5-Trimethoxyamphetamine hydrochloride (TMA) | < 1 |
| Urochloralic acid | < 1 |
| Zaleplon | < 1 |
| Zolpidem tartrate | < 1 |

(continued)

| Compound | % Cross-reactivity |
|---|---|
| Zopiclone | < 1 |

The antibodies of the invention are also able to bind to an epitope of molecules of Formula IV in which $R_1$ and $R_2$ are independently methyl, ethyl or propyl. Two such molecules are butylone and pentylone (Table 2).

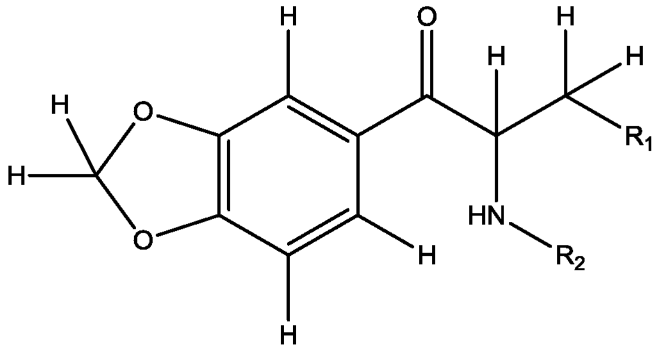

Formula IV

The invention further describes a kit comprising antibodies of the invention, the kit used to detect or determine molecules comprising either Formula II or Formula IV; preferably the kit is used to detect one or more of (RS)-1-(benzo[d][1,3]dioxol-5-yl)-2-(pyrrolidin-1-yl)butanone, (RS)-1-(2-naphthyl)-2-(pyrrolidin-1-yl)pentanone, (RS)-1-(4-methylphenyl)-2-(pyrrolidin-1-yl)butanone, (RS)-1-(benzo[d][1,3]dioxol-5-yl)-2-(pyrrolidin-1-yl)pentanone, (RS)-1-(benzo[d][1,3]dioxol-5-yl)-2-(pyrrolidin-1-yl)propanone, (RS)-1-(4-Methylphenyl)-2-(pyrrolidin-1-yl)hexanone, pyrovalerone, butylone and pentylone;

## Claims

**1.** A hapten or immunogen of formula I

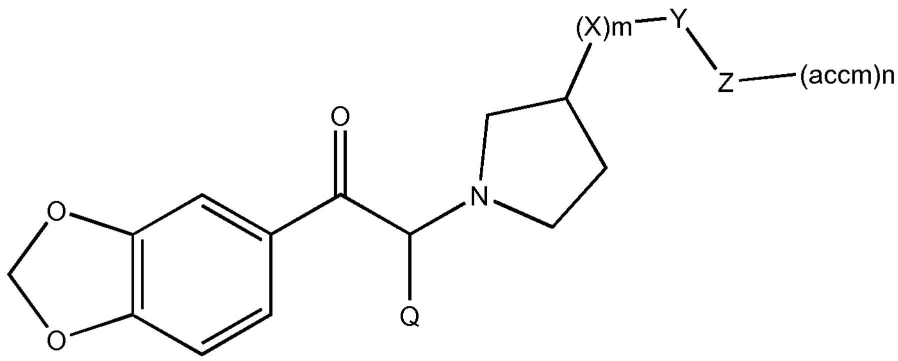

formula I

wherein,
for the hapten n=0; Q is $C_1$ - $C_4$ alkyl; X is N, O or S and m=0 or 1; Y is a substituted or unsubstituted $C_1$ - $C_{10}$, more preferably a $C_1$ - $C_6$, most preferably a $C_1$ - $C_3$ substituted or unsubstituted straight chain alkylene moiety; Z is selected from a carboxy, a dithiopyridyl, a maleimide, amino, hydroxyl, thiol or an aldehyde moiety;

for the immunogen n=1; Q is $C_1$ - $C_4$ alkyl; X is N, O or S and m=0 or 1; Y is a substituted or unsubstituted $C_1$ - $C_{10}$, more preferably a $C_1$ - $C_6$, most preferably a $C_1$ - $C_3$ substituted or unsubstituted straight chain alkylene moiety; Z, before connection to the accm, is selected from a carboxy, a dithiopyridyl, a maleimide, amino, hydroxyl, thiol or an aldehyde moiety; the accm is an antigenicity-conferring carrier material comprising poly(amino acid) segments.

2. The hapten or immunogen of Claim 1 in which for the hapten X is O, Y is -C(O)-$CH_2$-$CH_2$-, Z is carboxy or amino and Q is ethyl or propyl; and for the immunogen X is O, Y is -C(O)-$CH_2$-$CH_2$-, Z is carboxy or amino, Q is ethyl or propyl and the accm is BTG, BSA or KLH.

3. An antibody raised to an immunogen of either of the preceding claims the antibody further **characterised by** being able to bind to at least one structural epitope of a molecule of formula II

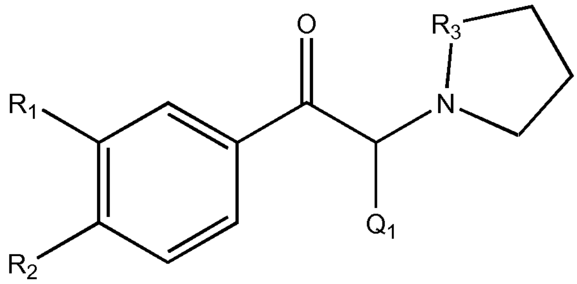

formula II

wherein $Q_1$ is $C_1$ - $C_4$ alkyl; $R_3$ is -$CH_2$- or -C(O)- ; $R_1$ and $R_2$ are H, $C_1$ - $C_4$ alkyl, $C_1$ - $C_4$ alkoxy, hydroxy substituted $C_1$ - $C_4$ alkyl, carboxy or hydroxyl, or together form substituted or unsubstituted

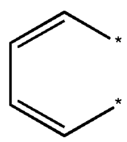

or

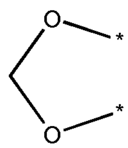

4. The antibody of Claim 3 which is able to bind to at least one structural epitope of any of the molecules (*RS*)-1-(benzo[*d*][1,3]dioxol-5-yl)-2-(pyrrolidin-1-yl)butanone, (*RS*)-1-(2-naphthyl)-2-(pyrrolidin-1-yl)pentanone, (*RS*)-1-(4-methylphenyl)-2-(pyrrolidin-1-yl)butanone, (*RS*)-1-(benzo[d][1,3]dioxol-5-yl)-2-(pyrrolidin-1-yl)pentanone or (*RS*)-1-(benzo[*d*][1,3]dioxol-5-yl)-2-(pyrrolidin-1-yl)propanone.

5. A method of detecting or determining one or more pyrrolidinophenones in an *in vitro* sample the method comprising contacting the sample with at least one detecting agent and at least one antibody of either of claims 3 or 4; detecting or determining the detecting agent(s); and deducing from a calibration curve the presence of, or amount of, pyrrolidinophenones in the sample.

6. The method of Claim 5 in which the pyrrolidinophenones to be detected or determined are one or more of (RS)-1-(benzo[d][1,3]dioxol-5-yl)-2-(pyrrolidin-1-yl)butanone, (RS)-1-(2-naphthyl)-2-(pyrrolidin-1-yl)pentanone, (*RS*)-1-(4-methylphenyl)-2-(pyrrolidin-1-yl)butanone, (*RS*)-41-(benzo[*d*][1,3]dioxol-5-yl)-2-(pyrrolidin-1-yl)pentanone and (*RS*)-1-(benzo[*d*][1,3]dioxol-5-yl)-2-(pyrrolidin-1-yl)propanone.

7. A compound of formula III

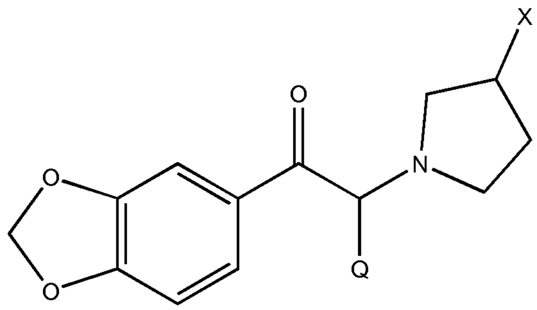

formula III

in which X is $NH_2$, OH or SH and Q is $C_1$ - $C_4$ alkyl.

**8.** The compound of Claim 7 in which X is OH and Q is ethyl or propyl.

**9.** A kit for detecting or determining pyrrolidinophenones the kit comprising at least one antibody of either of claims 3 or 4.

**10.** The kit of Claim 9 which detects or determines one or more of (RS)-1-(benzo[*d*][1,3]dioxol-5-yl)-2-(pyrrolidin-1-yl)butanone, (*RS*)-1-(2-naphthyl)-2-(pyrrolidin-1-yl)pentanone, (*RS*)-1-(4-methylphenyl)-2-(pyrrolidin-1-yl)butanone, (*RS*)-1-(benzo[*d*][1,3]dioxol-5-yl)-2-(pyrrolidin-1-yl)pentanone and *(RS)*-1-(benzo[*d*][1,3]dioxol-5-yl)-2-(pyrrolidin-1-yl)propanone.

**11.** An antibody raised to an immunogen of either of Claims 1 or 2 the antibody further **characterized by** being able to bind to at least one structural epitope of a molecule of formula IV wherein $R_1$ and $R_2$ are independently methyl, ethyl or propyl.

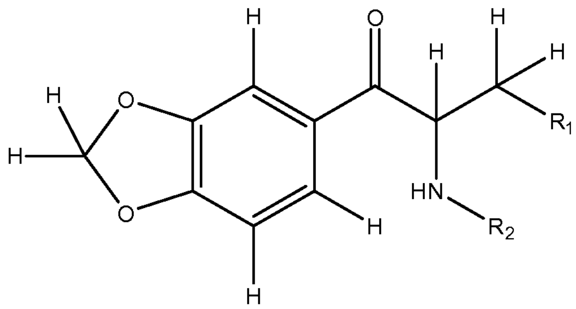

formula IV

**12.** The antibody of Claim 11 in which $R_2$ is methyl and $R_1$ is methyl or ethyl.

**13.** A kit for detecting or determining molecules comprising structures of either formula II of Claim 3 or formula IV of Claim 11 the kit comprising at least one antibody derived from an immunogen of either of Claims 1 or 2.

**14.** The kit of Claim 13 for detecting or determining at least one or more of (*RS*)-1-( benzo[*d*][1,3]dioxol-5-yl)-2-(pyrrolidin-1-yl)butanone, (*RS*)-1-(2-naphthyl)-2-(pyrrolidin-1-yl)pentanone, (*RS*)-1-(4-methylphenyl)-2-(pyrrolidin-1-yl)butanone, (*RS*)-1-(benzo[*d*][1,3]dioxol-5-yl)-2-(pyrrolidin-1-yl)pentanone, (*RS*)-1-(benzo[*d*][1,3]dioxol-5-yl)-2-(pyrrolidin-1-yl)propanone, (*RS*)-1-(4-Methylphenyl)-2-(pyrrolidin-1-yl)hexanone, pyrovalerone, butylone and pentylone.

**Patentansprüche**

**1.** Hapten oder Immunogen mit der Formel I

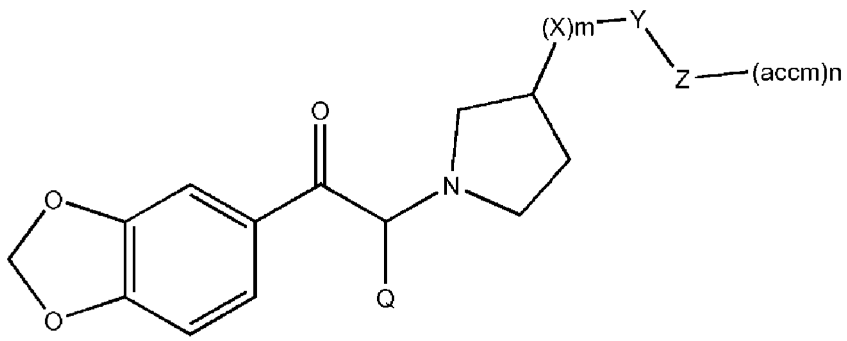

Formel I

wobei

für das Hapten n=0 ist; Q $C_1$-$C_4$-Alkyl ist; X N, O oder S ist und m=0 oder 1 ist; Y ein substituierter oder unsubstituierter $C_1$-$C_{10}$-, stärker bevorzugt ein $C_1$-$C_6$-, am stärksten bevorzugt ein $C_1$-$C_3$-substituierter oder unsubstituierter geradkettiger Alkylenanteil ist; Z ausgewählt ist aus einem Carboxy-, Dithiopyridyl-, Maleimid-, Amino-, Hydroxyl-, Thiol- oder einem Aldehydanteil;

für das Immunogen n=1 ist; Q $C_1$-$C_4$-Alkyl ist; X N, O oder S ist und m=0 oder 1 ist; Y ein substituierter oder unsubstituierter $C_1$-$C_{10}$-, stärker bevorzugt ein $C_1$-$C_6$-, am stärksten bevorzugt ein $C_1$-$C_3$-substituierter oder unsubstituierter geradkettiger Alkylenanteil ist; Z vor der Bindung an das accm ausgewählt ist aus einer Gruppe bestehend aus einem Carboxy-, Dithiopyridyl-, Maleimid-, Amino-, Hydroxyl-, Thiol- oder einem Aldehydanteil; und das accm ein Antigenität verleihendes Trägermaterial ist, das Poly(Aminosäure)-Segmente umfasst.

**2.** Hapten oder Immunogen nach Anspruch 1 in wobei für das Hapten X O ist, Y - C(O)-$CH_2$-$CH_2$- ist, Z Carboxy oder Amino ist und Q Ethyl oder Propyl ist; und für das Immunogen X O ist, Y -C(O)-$CH_2$-$CH_2$- ist, Z Carboxy oder Amino ist, Q Ethyl oder Propyl ist und das accm BTG, BSA oder KLH ist.

**3.** Antikörper gegen ein Immunogen nach einem der vorhergehenden Ansprüche, wobei der Antikörper ferner **dadurch gekennzeichnet ist, dass** er sich an wenigstens ein strukturelles Epitop eines Moleküls mit der Formel II binden kann

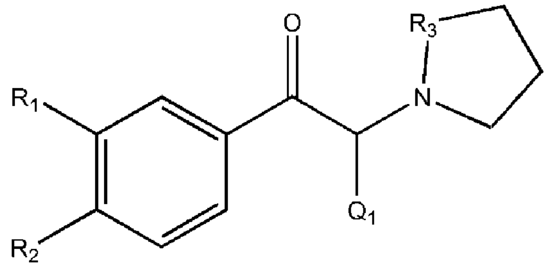

Formel II

wobei $Q_1$ $C_1$-$C_4$-Alkyl ist; $R_3$ -$CH_2$- oder -C(O)- ist; $R_1$ und $R_2$ jeweils H, $C_1$-$C_4$-Alkyl, $C_1$-$C_4$-Alkoxy, hydroxysubstituiertes $C_1$-$C_4$-Alkyl, Carboxy oder Hydroxyl sind, oder gemeinsam substituiertes oder unsubstituiertes

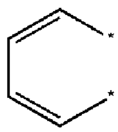

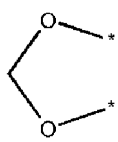

oder

ausbilden.

4. Antikörper nach Anspruch 3, der in der Lage ist, sich an wenigstens ein strukturelles Epitop eines der Moleküle (*RS*)-1-(benzo[*d*][1,3]dioxol-5-yl)-2-(pyrrolidin-1-yl)butanon, (RS)-1-(2-naphthyl)-2-(pyrrolidin-1-yl)pentanon, (RS)-1-(4-methylphenyl)-2-(pyrrolidin-1-yl)butanon, (*RS*)-1-(benzo[*d*][1,3]dioxol-5-yl)-2-(pyrrolidin-1-yl)pentanon oder (RS)-1-(benzo[*d*][1,3]dioxol-5-yl)-2-(pyrrolidin-1-yl)propanon zu binden.

5. Verfahren zum Erfassen oder Bestimmen eines oder mehrerer Pyrrolidinophenone in einer Probe *in vitro,* wobei das Verfahren Folgendes umfasst: In-Berührung-Bringen der Probe mit wenigstens einem Prüfmittel und wenigstens einem Antikörper nach einem der Ansprüche 3 oder 4; Erfassen oder Bestimmen des / der Prüfmittel(s); und Ableiten der Anwesenheit oder der Menge von Pyrrolidinophenonen in der Probe aus einer Kalibrierungskurve.

6. Verfahren nach Anspruch 5, in dem die zu ermittelnden oder zu bestimmenden Pyrrolidinophenone eines oder mehrere der Folgenden sind: (*RS*)-1-(benzo[*d*][1,3]dioxol-5-yl)-2-(pyrrolidin-1-yl)butanon, (*RS*)-1-(2-naphthyl)-2-(pyrrolidin-1-yl)pentanon, (RS)-1-(4-methylphenyl)-2-(pyrrolidin-1-yl)butanon, (*RS*)-1-(benzo[*d*][1,3]dioxol-5-yl)-2-(pyrrolidin-1-yl)pentanon und (*RS*)-1-(benzo[*d*][1,3]dioxol-5-yl)-2-(pyrrolidin-1-yl)propanon.

7. Verbindung mit der Formel III

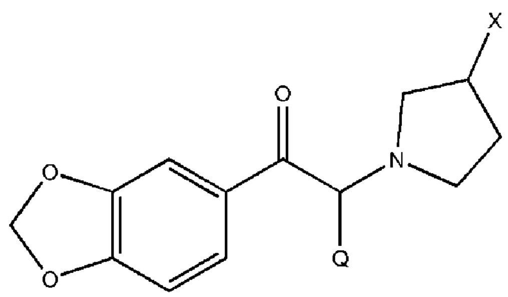

Formel III

in der X $NH_2$, OH oder SH ist und Q $C_1$-$C_4$-Alkyl ist.

8. Verbindung nach Anspruch 7 in der X OH ist und Q Ethyl oder Propyl ist.

9. Kit zum Erfassen oder zum Bestimmen von Pyrrolidinophenonen, wobei das Kit wenigstens einen Antikörper nach einem der Ansprüche 3 oder 4 aufweist.

10. Kit nach Anspruch 9, welches eines oder mehrere der Folgenden erfasst oder bestimmt: (*RS*)-1-(benzo[*d*][1,3]dioxol-5-yl)-2-(pyrrolidin-1-yl)butanon, (*RS*)-1-(2-naphthyl)-2-(pyrrolidin-1-yl)pentanon, (*RS*)-1-(4-methylphenyl)-2-(pyrrolidin-1-yl)butanon, *(RS)-1*-(benzo[*d*][1,3]dioxol-5-yl)-2-(pyrrolidin-1-yl)pentanon und (*RS*)-1-(benzo[*d*][1,3]dioxol-5-yl)-2-(pyrrolidin-1-yl)propanon.

11. Antikörper gegen ein Immunogen nach einem der Ansprüche 1 und 2, wobei der Antikörper ferner **dadurch gekennzeichnet ist, dass** er sich an wenigstens ein strukturelles Epitop eines Moleküls mit der Formel IV bindet, wobei $R_1$ und $R_2$ unabhängig voneinander Methyl, Ethyl oder Propyl sind.

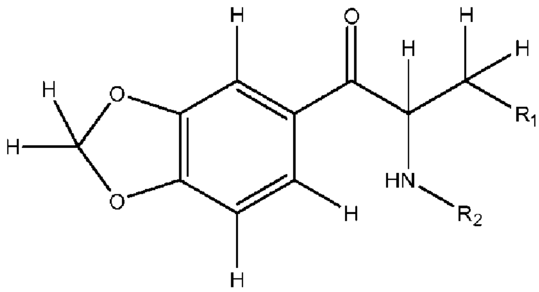

Formel IV

12. Antikörper nach Anspruch 11, in dem $R_2$ Methyl ist und $R_1$ Methyl oder Ethyl ist.

13. Kit zum Erfassen oder Bestimmen von Molekülen, die Strukturen mit der Formel II aus Anspruch 3 oder der Formel IV aus Anspruch 11 umfassen, wobei das Kit wenigstens einen Antikörper umfasst, der aus einem Immunogen nach einem der Ansprüche 1 und 2 abgeleitet ist.

14. Kit nach Anspruch 13 zum Erfassen oder Bestimmen wenigstens eines oder mehrerer der Folgenden: (*RS*)-1-(benzo[*d*][1,3]dioxol-5-yl)-2-(pyrrolidin-1-yl)butanon, (RS)-1-(2-naphthyl)-2-(pyrrolidin-1-yl)pentanon, (*RS*)-1-(4-methylphenyl)-2-(pyrrolidin-1-yl)butanon, (*RS*)-1-(benzo[*d*][1,3]dioxol-5-yl)-2-(pyrrolidin-1-yl)pentanon, (*RS*)-1-(benzo[*d*][1,3]dioxol-5-yl)-2-(pyrrolidin-1-yl)propanon, (*RS*)-1-(4-methylphenyl)-2-(pyrrolidin-1-yl)hexanon, Pyrovaleron, Butylon und Pentylon.

**Revendications**

1. Haptène ou immunogène de formule I

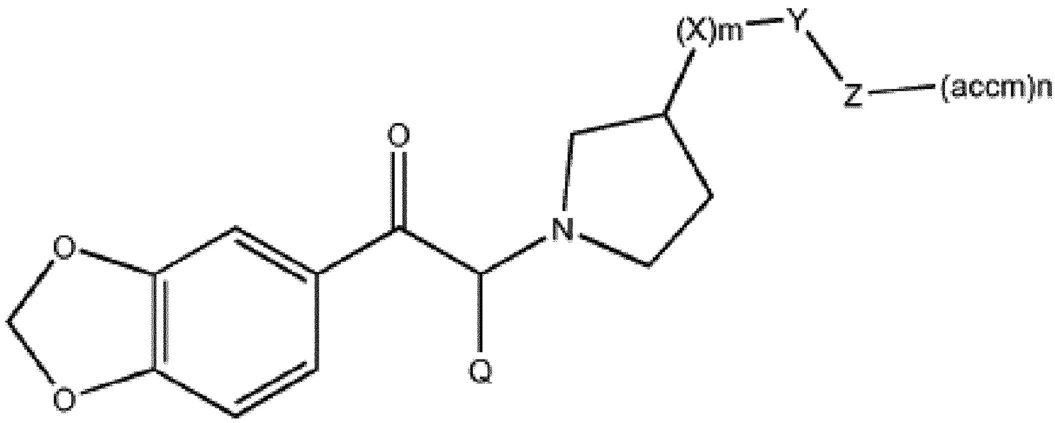

formule I

où
pour l'haptène n = 0 ; Q est un groupe alkyle en $C_1$ à $C_4$ ; X est N, O ou S et m = 0 ou 1 ; Y est un fragment alkylène à chaîne linéaire substitué ou non substitué en $C_1$ à $C_{10}$, plus préférablement en $C_1$ à $C_6$, le plus préférablement en $C_1$ à $C_3$ ; Z est choisi parmi un fragment carboxy, dithiopyridyle, maléimide, amino, hydroxyle, thiol ou aldéhyde ;
pour l'immunogène n = 1 ; Q est un groupe alkyle en $C_1$ à $C_4$ ; X est N, O ou S et m = 0 ou 1 ; Y est un fragment alkylène à chaîne linéaire substitué ou non substitué en $C_1$ à $C_{10}$, plus préférablement en $C_1$ à $C_6$, le plus préférablement en $C_1$ à $C_3$ ; Z, avant d'être relié à l'accm, est choisi parmi un fragment carboxy, dithiopyridyle, maléimide, amino, hydroxyle, thiol ou aldéhyde ; l'accm est un matériau vecteur conférant une antigénicité comprenant des segments poly(acides aminés).

2. Haptène ou immunogène selon la revendication 1, où, pour l'haptène, X est O, Y est $-C(O)-CH_2-CH_2-$, Z est un

groupe carboxy ou amino et Q est un groupe éthyle ou propyle ; et pour l'immunogène, X est O, Y est -C(O)-$CH_2$-$CH_2$-, Z est un groupe carboxy ou amino, Q est un groupe éthyle ou propyle et l'accm est BTG, BSA ou KLH.

3. Anticorps dirigé contre un immunogène selon l'une des revendications précédentes, l'anticorps étant en outre **caractérisé en ce qu'**il est capable de se lier à au moins un épitope structurel d'une molécule de formule II

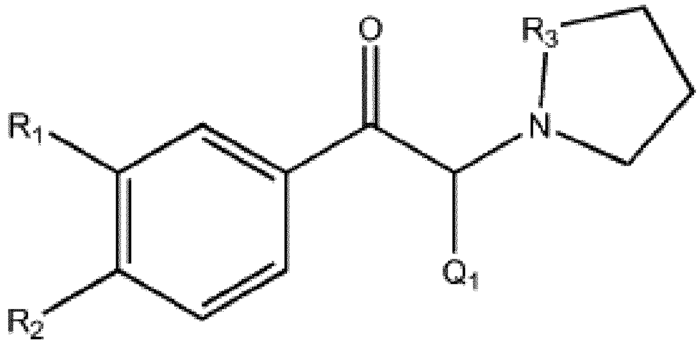

formule II

où $Q_1$ est un groupe alkyle en $C_1$ à $C_4$ ; $R_3$ est -$CH_2$- ou -C(O)- ; $R_1$ et $R_2$ sont H, un groupe alkyle en $C_1$ à $C_4$, un groupe alcoxy en $C_1$ à $C_4$, un groupe alkyle en $C_1$ à $C_4$ substitué par un groupe hydroxy, un groupe carboxy ou un groupe hydroxyle, ou forment conjointement des groupes substitués ou non substitués.

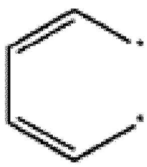

ou

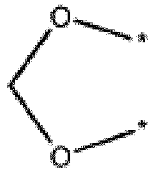

4. Anticorps selon la revendication 3, qui est capable de se lier à au moins un épitope structurel de l'une quelconque des molécules (*RS*)-1-(benzo[*d*][1,3]dioxol-5-yl)-2-(pyrrolidin-1-yl)butanone, (*RS*)-1-(2-naphtyl)-2-(pyrrolidin-1-yl)pentanone, (RS)-1-(4-méthylphényl)-2-(pyrrolidin-1-yl)butanone, (*RS*)-1-(benzo[*d*][1,3]dioxol-5-yl)-2-(pyrrolidin-1-yl)pentanone ou (*RS*)-1-(benzo[*d*][1,3]dioxol-5-yl)-2-(pyrrolidin-1-yl)propanone.

5. Procédé de détection ou de détermination d'une ou de plusieurs pyrrolidinophénones dans un échantillon *in vitro,* le procédé comprenant les étapes consistant à mettre en contact l'échantillon avec au moins un agent de détection et au moins un anticorps selon l'une des revendications 3 ou 4 ; détecter ou déterminer le ou les agent(s) de détection ; et déduire d'après une courbe d'étalonnage la présence ou la quantité de pyrrolidinophénones dans l'échantillon.

6. Procédé selon la revendication 5, où les pyrrolidinophénones à détecter ou à déterminer sont une ou plusieurs de la (*RS*)-1-(benzo[*d*][1,3]dioxol-5-yl)-2-(pyrrolidin-1-yl)butanone, (*RS*)-1-(2-naphtyl)-2-(pyrrolidin-1-yl)pentanone, (*RS*)-1-(4-méthylphényl)-2-(pyrrolidin-1-yl)butanone, (*RS*)-1-(benzo[*d*][1,3]dioxol-5-yl)-2-(pyrrolidin-1-yl)pentanone et (*RS*)-1-(benzo[*d*][1,3]dioxol-5-yl)-2-(pyrrolidin-1-yl)propanone.

7. Composé de formule III

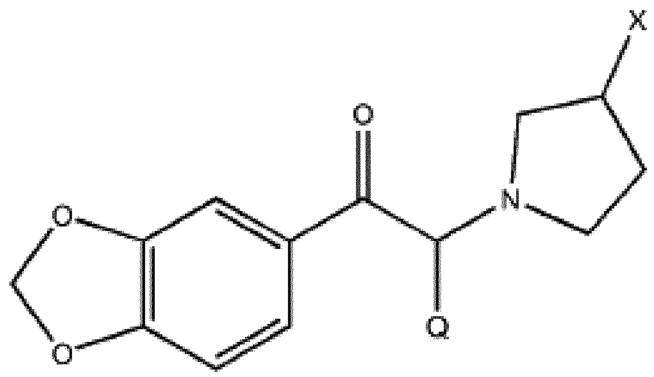

formule III

où X est $NH_2$, OH ou SH et Q est un groupe alkyle en $C_1$ à $C_4$.

**8.** Composé selon la revendication 7, où X est OH et Q est un groupe éthyle ou propyle.

**9.** Kit de détection ou de détermination de pyrrolidinophénones, le kit comprenant au moins un anticorps selon l'une des revendications 3 ou 4.

**10.** Kit selon la revendication 9, qui permet de détecter ou de déterminer une ou plusieurs de la (*RS*)-1-(benzo[*d*][1,3]dioxol-5-yl)-2-(pyrrolidin-1-yl)butanone, (*RS*)-1-(2-naphtyl)-2-(pyrrolidin-1-yl)pentanone, (*RS*)-1-(4-méthylphényl)-2-(pyrrolidin-1-yl)butanone, (*RS*)-1-(benzo[*d*][1,3]dioxol-5-yl)-2-(pyrrolidin-1-yl)pentanone et *RS*)-1-(benzo[*d*][1,3]dioxol-5-yl)-2-(pyrrolidin-1-yl)propanone.

**11.** Anticorps dirigé contre un immunogène selon l'une des revendications 1 ou 2, l'anticorps étant en outre **caractérisé en ce qu'**il est capable de se lier à au moins un épitope structurel d'une molécule de formule IV, où $R_1$ et $R_2$ sont indépendamment un groupe méthyle, éthyle ou propyle.

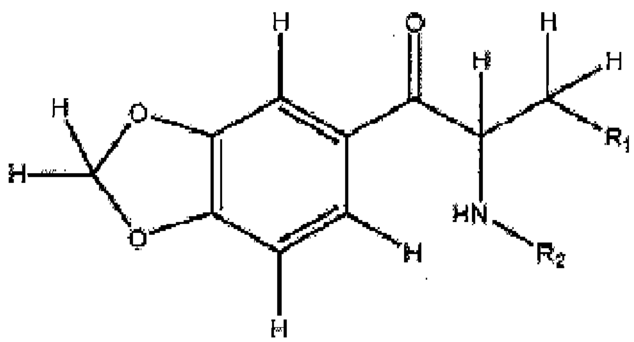

formule IV

**12.** Anticorps selon la revendication 11, où $R_2$ est un groupe méthyle et $R_1$ est un groupe méthyle ou éthyle.

**13.** Kit de détection ou de détermination de molécules comprenant des structures de formule II selon la revendication 3 ou de formule IV selon la revendication 11, le kit comprenant au moins un anticorps dérivé d'un immunogène selon l'une des revendications 1 ou 2.

**14.** Kit selon la revendication 13, qui permet de détecter ou de déterminer une ou plusieurs de la (*RS*)-1-(benzo[*d*][1,3]dioxol-5-yl)-2-(pyrrolidin-1-yl)butanone, (*RS*)-1-(2-naphtyl)-2-(pyrrolidin-1-yl)pentanone, (*RS*)-1-(4-méthylphényl)-2-(pyrrolidin-1-yl)butanone, (*RS*)-1-(benzo[*d*][1,3]dioxol-5-yl)-2-(pyrrolidin-1-yl)pentanone, (*RS*)-1-(benzo[*d*][1,3]dioxol-5-yl)-2-(pyrrolidin-1-yl)propanone, (RS)-1-(4-méthylphényl)-2-(pyrrolidin-1-yl)hexanone, pyrovalérone, butylone et pentylone.

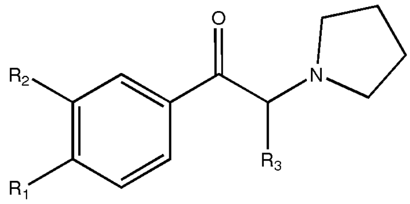

| Molecule | R1 | R2 | R3 |
|---|---|---|---|
| 1. | H | H | $-CH_3$ |
| 2. | H | H | $-CH_2-CH_3$ |
| 3. | H | H | $-(CH_2)_2-CH_3$ |
| 4. | H | H | $-(CH_2)_3-CH_3$ |
| 5. | $-CH_3$ | H | $-CH_3$ |
| 6. | $-CH_3$ | H | $-CH_2-CH_3$ |
| 7. | $-CH_3$ | H | $-(CH_2)_2-CH_3$ |
| 8. | $-CH_3$ | H | $-(CH_2)_3-CH_3$ |
| 9. | $-O-CH_3$ | H | $-CH_3$ |
| 10. | -OH | -OH | $-CH_3$ |
| 11. | -OH | $-O-CH_3$ | $-CH_3$ |
| 12 | $-O-CH_3$ | -OH | $-CH_3$ |
| 13. | -OH | -OH | $-CH_2-CH_3$ |
| 14. | -OH | $-O-CH_3$ | $-CH_2-CH_3$ |
| 15. | $-O-CH_3$ | -OH | $-CH_2-CH_3$ |
| 16. | -OH | -OH | $-(CH_2)_2-CH_3$ |
| 17. | -OH | $-O-CH_3$ | $-(CH_2)_2-CH_3$ |
| 18. | $-O-CH_3$ | -OH | $-(CH_2)_2-CH_3$ |
| 19. | -OH | -OH | $-(CH_2)_3-CH_3$ |
| 20. | -OH | $-O-CH_3$ | $-(CH_2)_3-CH_3$ |
| 21. | $-O-CH_3$ | -OH | $-(CH_2)_3-CH_3$ |
| 22. | 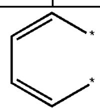 | | $-(CH_2)_2-CH_3$ |
| 23. | 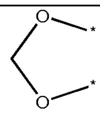 | | $-CH_3$ |
| 24. | 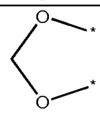 | | $-CH_2-CH_3$ |
| 25. | 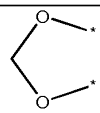 | | $-(CH_2)_2-CH_3$ |

**Figure 1**

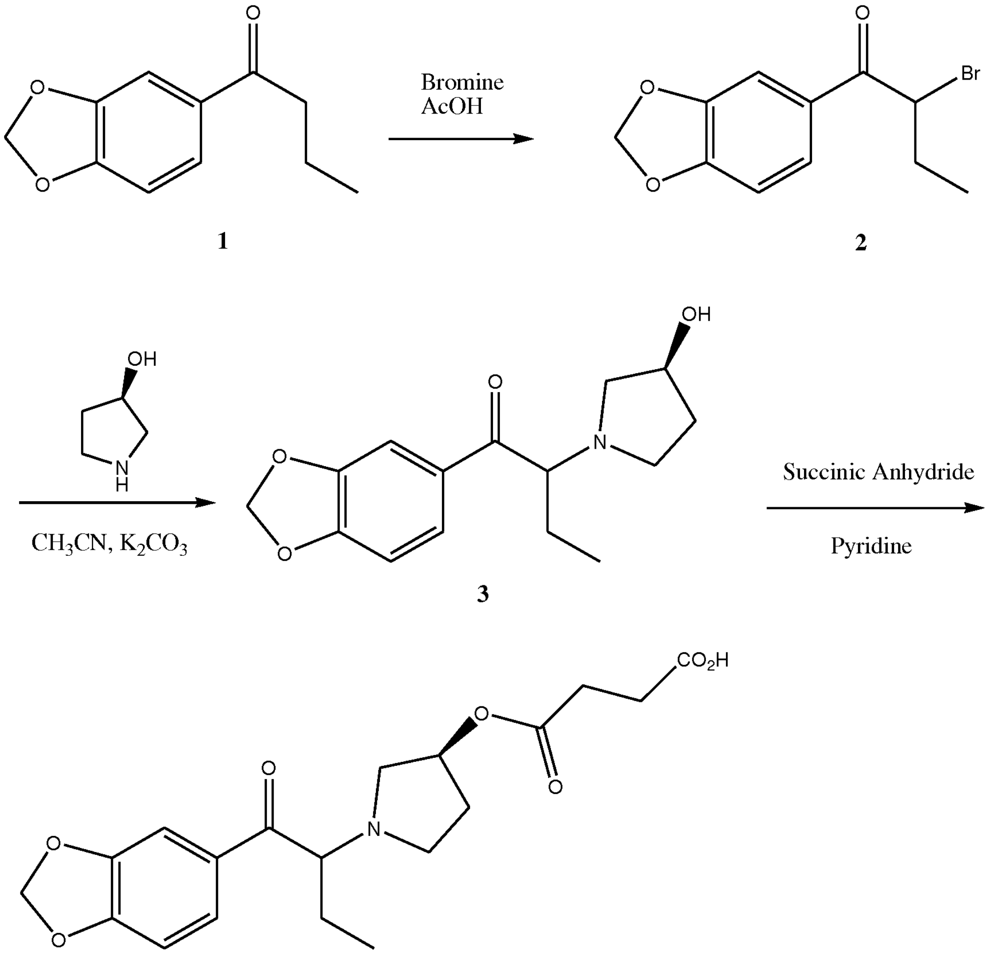


1

2

3

**Hapten - A**

**Figure 2**

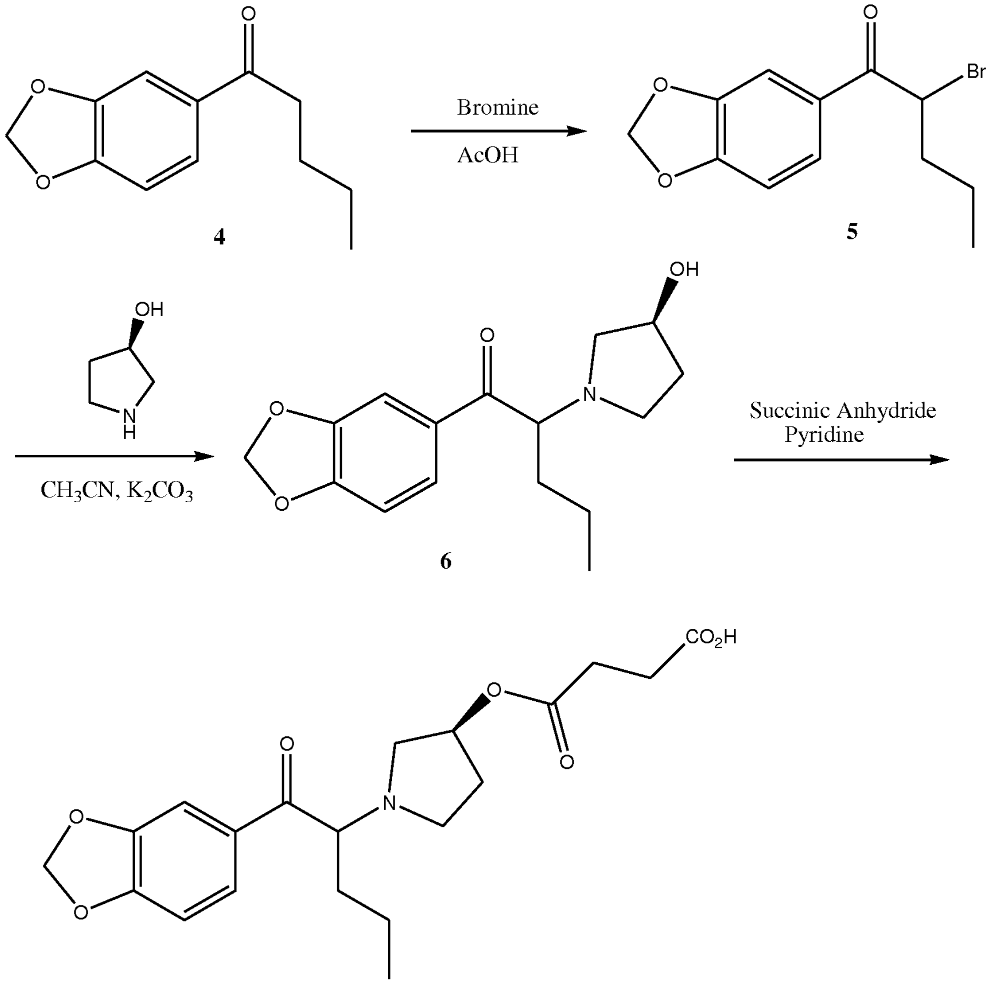


4

5

6

Hapten - B

**Figure 3**

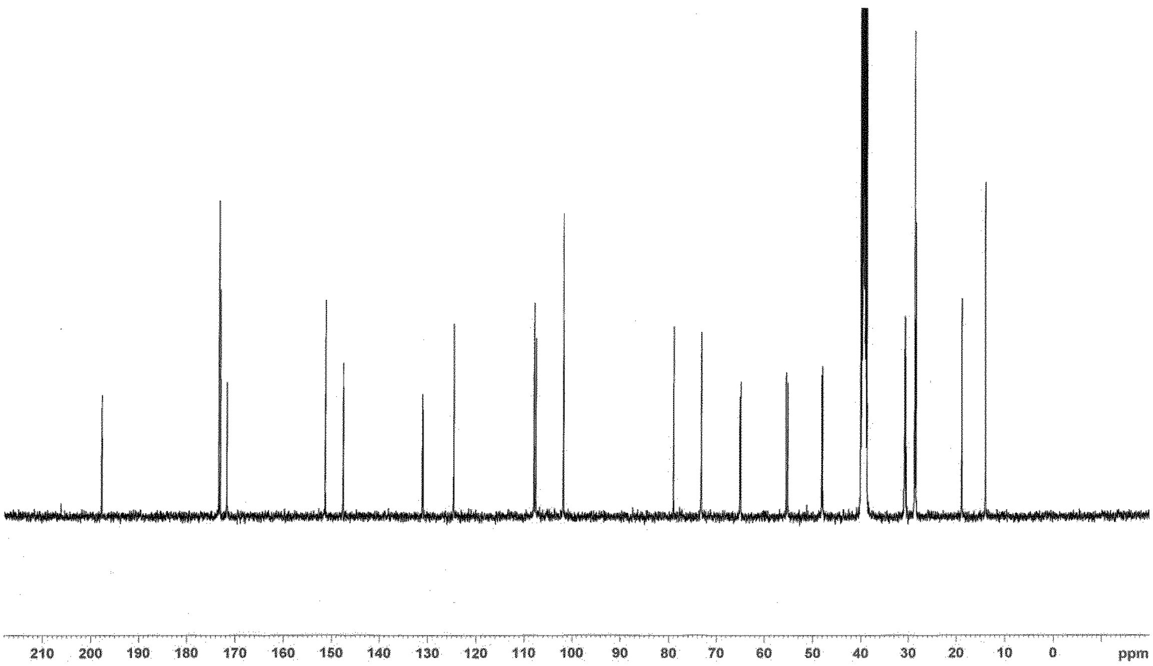
210
200
190
180
170
160
150
140
130
120
110
100
90
80
70
60
50
40
30
20
10
0
ppm

Figure 4

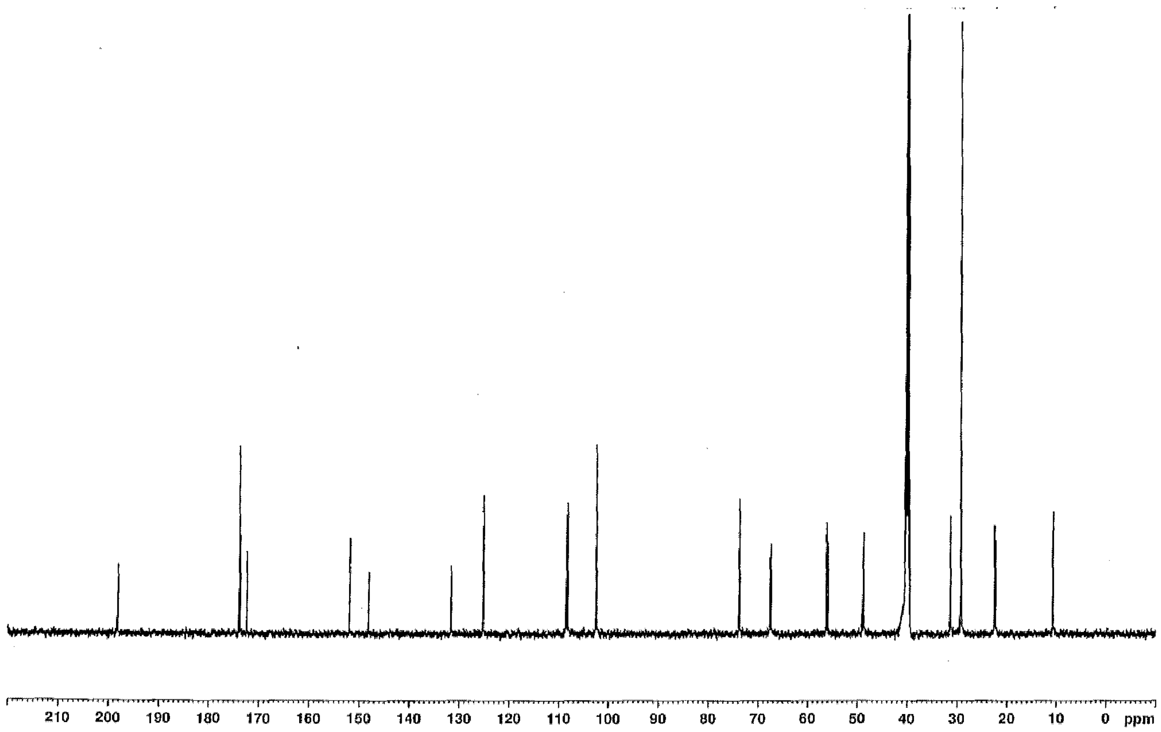
210 200 190 180 170 160 150 140 130 120 110 100 90 80 70 60 50 40 30 20 10 0 ppm

Figure 5

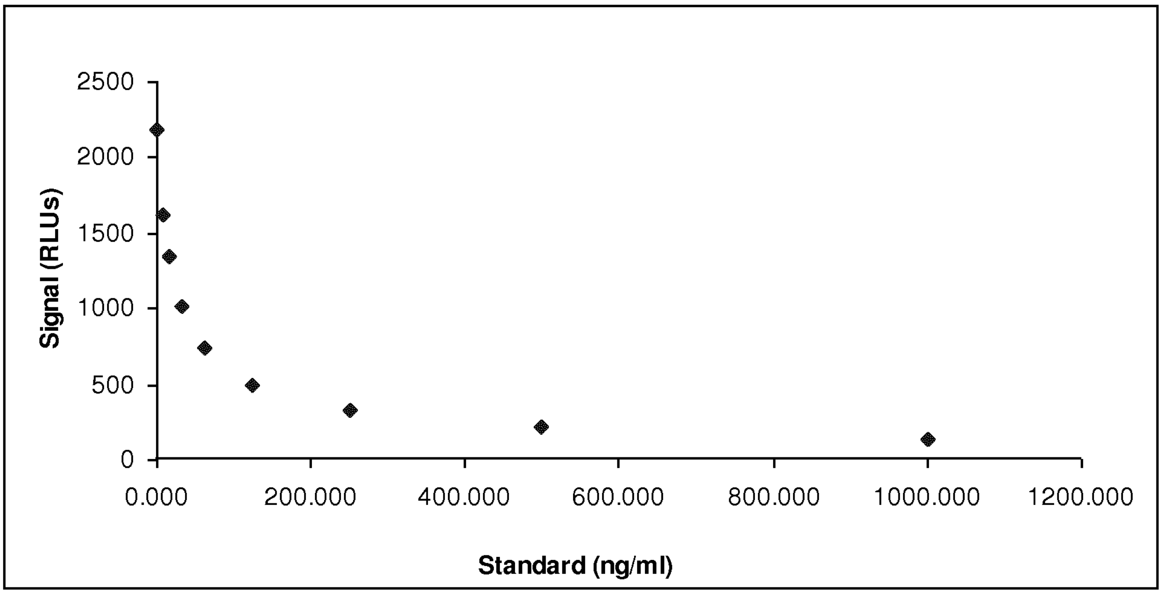
2500
2000
1500
1000
500
0
Signal (RLUs)
0.000
200.000
400.000
600.000
800.000
1000.000
1200.000
Standard (ng/ml)

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **YOHANNAN J.C. ; BOZENKO J.S.** *Microgram J.,* 2010, vol. 1, 12-15
- **STRANO-ROSSI S. et al.** *Rapid Commun. Mass Spectrom.,* 2010, vol. 24, 2706-2714
- **FITZGERALD S.P. et al.** *Clin. Chem.,* 2005, vol. 51, 1165-1176